(19) Europäisches Patentamt
European Patent Office
Office européen des brevets

(11) Numéro de publication: **0 223 890 B1**

(12) # FASCICULE DE BREVET EUROPEEN

(45) Date de publication du fascicule du brevet: **22.11.90**

(51) Int. Cl.[5]: **C07D 207/34**, A61K 31/40

(21) Numéro de dépôt: **85402270.4**

(22) Date de dépôt: **22.11.85**

(54) **Nouveaux dérivés aroylés du pyrrole, leur obtention et leur emploi en thérapeutique immunologique.**

(43) Date de publication de la demande:
**03.06.87 Bulletin 87/23**

(45) Mention de la délivrance du brevet:
**22.11.90 Bulletin 90/47**

(84) Etats contractants désignés:
**AT BE CH DE FR GB IT LI LU NL SE**

(56) Documents cités:
**EP-A- 0 001 534**
**EP-A- 0 167 426**

**E. Schröder et al, Pharmazeutische Chemie, Verlag G. Thieme, 1982, Seiten 400-402**

(73) Titulaire: **Albert ROLLAND S.A. Société dite, 49, Rue St-André-des-Arts, F-75006 Paris(FR)**

(72) Inventeur: **Labaune, Jean-Pierre, 10, rue des Grandes Cèdres, Moulignon F-77130 Ponthierry(FR)**
Inventeur: **Bessin, Pierre, 1, allée Bossuet, F-91380 Chilly-Mazarin(FR)**

(74) Mandataire: **Burtin, Jean-François, Cabinet GEFIB 55 Rue Boissonade, F-75014 Paris(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 223 890 B1**

ACTORUM AG

## Description

La présente invention a pour objet des nouveaux dérivés aroylés du pyrrole. Elle a plus particulièrement pour objet des dérivés 4-aroylés de l'acide pyrrolyl-2-carboxylique.

Elle a spécifiquement pour objet de nouveaux dérivés naphtoylés de l'acide pyrrolyl-2-carboxylique répondant à la formule générale (I):

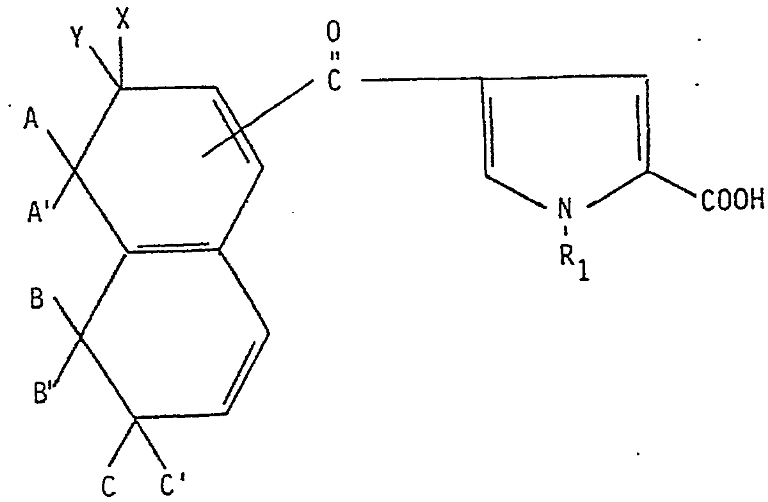

(I)

dans laquelle:
$R_1$ représente un radical méthyle, un radical phényle ou de l'hydrogène;
X est de l'hydrogène, un hydroxyle ou le groupe méthylsulfinyle;
Y est de l'hydrogène ou forme avec A' une double liaison;
A est de l'hydrogène ou un hydroxyle;
A' est de l'hydrogène ou forme avec Y une double liaison;
B est de l'hydrogène ou un hydroxyle;
B' est de l'hydrogène ou forme avec C une double liaison;
C est de l'hydrogène ou forme avec B' une double liaison;
C' est de l'hydrogène, un hydroxyle ou le groupe méthylsulfinyle ($SOCH_3$);
avec la restriction que X, A, B et C' ne représentent pas simultanément de l'hydrogène lorsque A' forme avec Y une double liaison et lorsque B' forme avec C une double liaison.

Les composés selon l'invention existent sous forme d'acide ou sous forme de sels de ces acides comme par exemple un sel de métal alcalin, de métal alcalino-terreux, de fer, d'aluminium ou de magnésium, un sel de base organique comme une alcoylamine, une dialcoylamine, une arylalcoylamine, une trialcoylamine, une amine cyclanique, un amino-acide basique, un amino-alcanol, une base cyclique comme le dihydropyridine, la lutidine ou la collidine.

Les composés de formule générale I dans laquelle X est un hydroxyle ou un groupe méthylsulfinyle et/ou dans laquelle B est un hydroxyle et B' de l'hydrogène et/ou dans laquelle C' est un hydroxyle ou un groupe méthylsulfinyle et C est de l'hydrogène, présentent de 1 à 3 centres d'asymétrie. Ils peuvent de ce fait exister sous forme de diastéréoisomères que l'on peut séparer par de méthodes chimiques, physiques ou enzymatiques. Les diastéréo-isomères peuvent ensuite être dédoublés en leurs isomères optiques.

Les diastéréo-isomères et les isomères optiques font partie de l'invention.

En fonction de la signification des différents substituants, on peut distinguer trois sous-groupes de composés également intéressants:

1) les dérivés 4-naphtoylés de formule générale $I_A$:

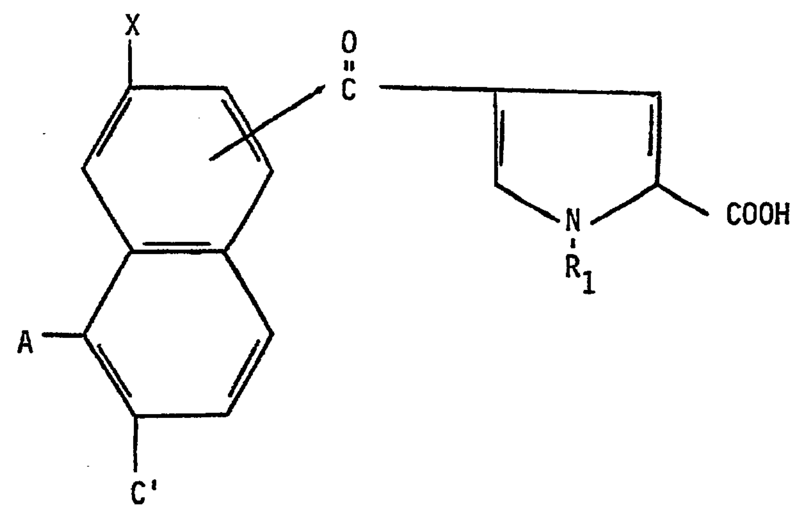

$(I_A)$

dans laquelle:
X est un groupe méthylsulfinyle et C′ est de l'hydrogène;
ou X est de l'hydrogène et C′ est un groupe méthylsulfinyle;
A est de l'hydrogène ou un hydroxyle; et
$R_1$ est défini comme précédemment.

2) les dihydronaphtalènediols de formule générale $I_B$:

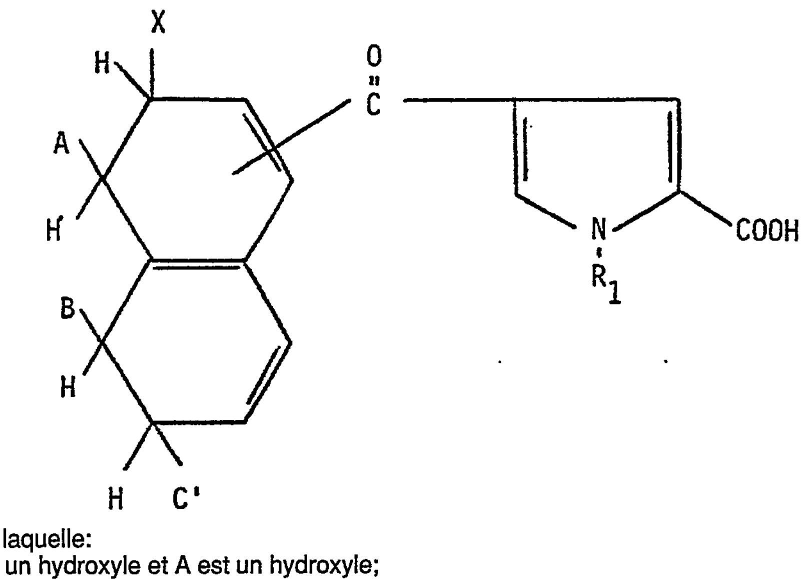

$(I_B)$

dans laquelle:
X est un hydroxyle et A est un hydroxyle;
B et C′ sont de l'hydrogène;
ou B et C′ sont chacun un hydroxyle et X et A sont de l'hydrogène; et
$R_1$ est défini comme précédemment.

3) les dérivés desalcoylés de formule générale $I_C$:

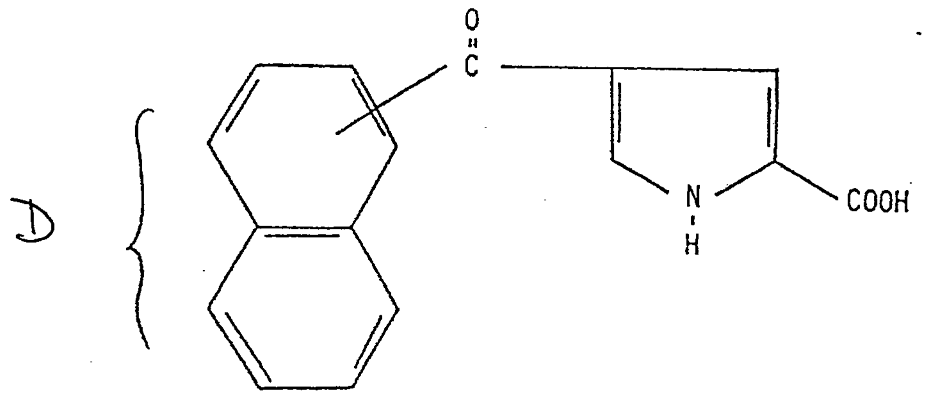

($I_C$)

dans laquelle D représente de l'hydrogène, un hydroxyle ou un méthylsulfinyle.

Dans toutes ces formules, le noyau naphtalénique est relié au carbonyle par une liaison en 1 ou en 2.

Les composés de formule générale I et leurs sels d'addition avec une base minérale ou organique se distinguent par d'intéressantes propriétés pharmacologiques. Ils manifestent en particulier des propriétés immunologiques et notamment des propriétés immuno-suppressives. Cependant, certains composés comme le dérivé (3-méthylsulfonylnaphtoyl-1) et les dérivés (3-hydroxy ou 5-hydroxy-naphtoyl-1)-pyrrolyl-carboxyliques montrent, selon les doses, des propriétés immuno-dépressives ou des propriétés immuno-stimulantes.

En raison de leur très faible toxicité, ils peuvent trouver un emploi en thérapeutique, notamment comme médicament des troubles de l'immunité.

En tant qu'agents immuno-suppresseurs, ils trouvent un emploi en médecine humaine ou animale dans le traitement des maladies auto-immunes comme le lupus, la polyarthrite rhumatoïde, le diabète juvénile insulinodépendant, les myopathies et certaines maladies rénales. Ils conviennent également pour prévenir ou empêcher les phénomènes de rejets lors d'implantation d'organe ou de greffe de tissus.

En tant que médicament immuno-stimulant, ils trouvent un emploi en médecine humaine ou animale dans le traitement des maladies chroniques comme la bronchite ou dans l'accélération des phénomènes de cicatrisation.

On connaissait déjà des acides naphtoylpyrrolyl carboxylique (Brevet français 2 405 245) éventuellement substitué par des radicaux alcoyle alcoxy ou halogène qui manifestent une activité uricosurique et qui de ce fait, trouvent un emploi dans le traitement des hyperuricémies pathologiques. Cette activité se manifeste chez l'animal à une dose relativement importante (50 mg/kg).

On connaissait également les propriétés immuno-dépressives des stéroides corticoides comme la Prednisone et la Prednisolone. Ces propriétés qui sont utilisés pour le traitement des maladies auto-immunes et pour la prévention du rejet de greffes. Toutefois, ce type d'action qui se manifeste ainsi à des doses très élevées est correlé à l'action anti-inflammatoire de ces composés.

L'effet des cortisoniques est en effet lié à une inhibition de la synthèse des protéines par interaction intracellulaire avec le noyau de la cellule. Les composés selon l'invention n'ont pas de propriétés anti-inflammatoires et n'interviennent ni dans la libération de l'acide arachidonique, ni dans la cascade de leurs métabolites. L'effet immuno-suppresseur ou immuno-activateur doit plutôt être attribué à un effet sur le matériel enzymatique des macrophages.

A cet effet, ils sont présentés sous forme de compositions pharmaceutiques renfermant, à titre de principe actif, au moins une composé de formule générale I ou un de ses sels d'addition avec une base minérale ou organique en association ou en mélange avec un excipient ou un véhicule inerte non toxique pharmaceutiquement acceptable.

L'excipient ou le véhicule est un de ceux qui conviennent pour l'administration par voie parentérale, digestive, rectale ou topique. On citera, à cet effet, les solutions aqueuses ou salines, les amidons, les celluloses, les alcoycelluloses, la carboxyméthylcellulose, le carboxyméthylamidon, les phosphates ou carbonates alcalino-terreux, le phosphate de magnésium, le beurre de cacao ou les stéarates de polyéthylèneglycol.

Les compositions pharmaceutiques selon l'invention sont présentées sous forme de composés nus ou enrobés, de dragées, de pilules, de gélules, de capsules molles, de solutés ou suspensions buvables, de solutés injectables répartis en ampoules, en flacons multidoses ou de seringues auto-injectables, de suppositoires ou de capsules rectales et sous forme de crèmes ou de préparation sous pression pour application muqueuse.

La posologie utile varie dans de larges proportions en fonction de la voie d'administration, du poids et de l'âge du sujet, de l'indication thérapeutique. En règle générale, la posologie unitaire s'échelonne de 25 à 1 500 mg, et la posologie journalière s'échelonne de 25 à 2 000 mg chez l'adulte.

Les composés de formule générale I sont obtenus aisément par les procédés usuels de la chimie organique au départ des acides aroylpyrrolyl-2-carboxyliques de formule générale II:

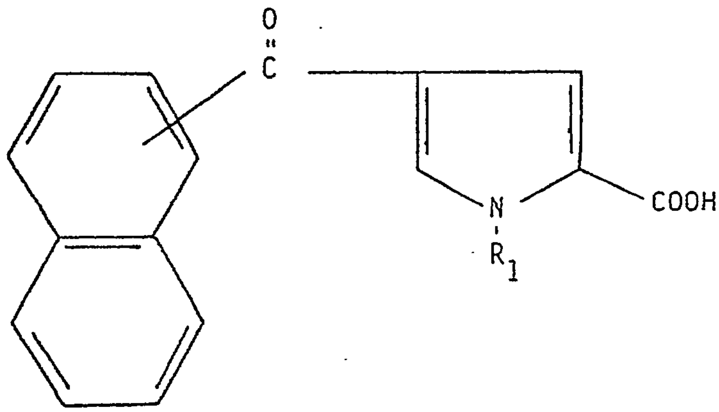

(II)

décrits dans le brevet français n° 2 405 246 au nom de la demanderesse.

Ils peuvent également être isolés dans les liquides biologiques comme par exemple l'urine par administration des acides aroylpyrrolyl-2-carboxyliques à des animaux et extraction par les méthodes physiques habituelles.

Les composés selon l'invention après purification sont caractérisés par les méthodes analytiques les plus modernes telle que le spectre de masse.

Les exemples suivants illustrent l'invention.

EXEMPLE I

## TABLEAU I

### VALEURS PRINCIPALES OBTENUES A PARTIR DES SPECTRES DE MASSE DE L'ACIDE N-METHYL-4-(NAPHTOYL-1)-PYRROLYL-2-CARBOXYLIQUE PRIS COMME SUBSTANCE DE REFERENCE

Impact électronique

| MASSE | % | |
|---|---|---|
| 279 | 18 | ——> pic moléculaire |
| 262 | 80 | ——> perte OH ou $H_2O$ (-17) |
| 234 | 80 | ——> perte COOH |
| 218 | 10 | |
| 205 | 10 | |
| 190 | 10 | |
| 165 | 20 | |
| 152 | 100 | ——> 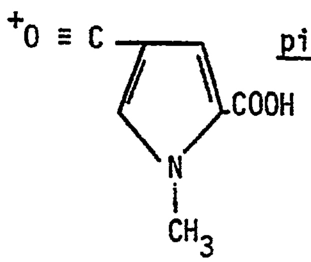 pic de base |
| 134 | 12 | |
| 127 | 60 | ——> naphtalène$^+$ |
| 108 | 10 | ——> 152 (-$CO_2$) |
| 58 | 90 | |

EXEMPLE II

Comparaison des caractéristiques spectrales de l'acide N-méthyl-4-(naphtoyl-1)-pyrrolyl-2-carboxylique (matière première) et de l'acide 4-(naphtoyl-1)pyrrolyl-2-carboxylique obtenu soit par synthèse, soit par extraction de l'urine.

TABLEAU II

SPECTRES RMN DANS LE DMSO DES ACIDES N-METHYL-4-(NAPHTOYL-1)-PYRROLYL-2-CARBOXYLIQUE ET 3-4-(NAPHTOYL-1)-PYRROLYL-2-CARBOXYLIQUE SYNTHETIQUE ET D'EXTRACTION

Acide N-méthyl-4-(naphtoyl-1)-pyrrolyl-2-carboxylique

Acide 4-(naphtoyl-1)-pyrrolyl-2-carboxylique de synthèse

Acide 4-(naphtoyl-1)-pyrrolyl-2-carboxylique obtenu par extraction biologique

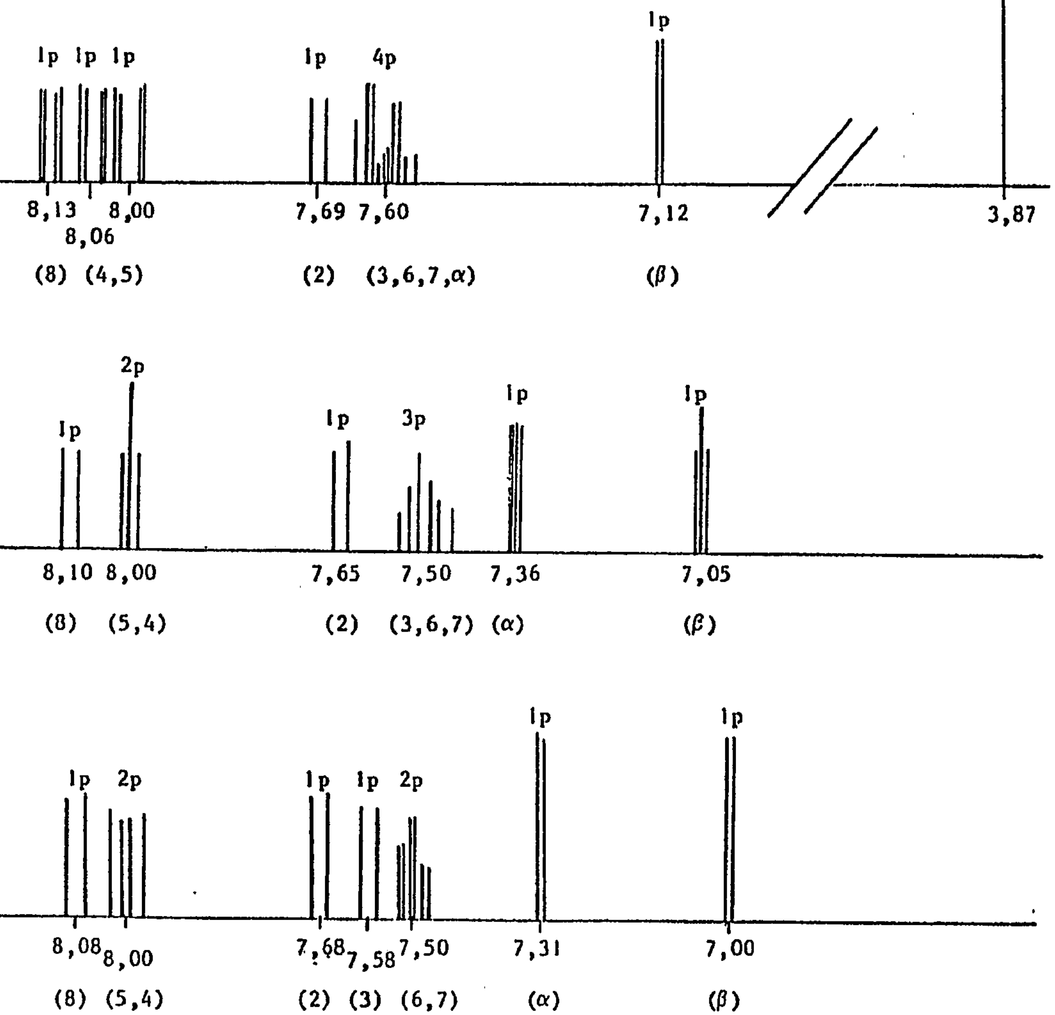

## TABLEAU III

### VALEURS PRINCIPALES OBTENUES A PARTIR DES SPECTRES DE MASSE DU DERIVE N-DEMETHYLE OBTENU PAR EXTRACTION

### Acide 4-(naphtoyl-1)-pyrrolyl-2-carboxylique

| Impact électronique | | | Ionisation chimique | | |
|---|---|---|---|---|---|
| 265 | 64% | — pic moléculaire | 282 | 19% | — ion molécul. + $NH_4^+$ |
| 246 | 22% | — perte $H_2O$ (-19) | 279 | 11% | |
| 230 | 5% | — (-35) | 266 | 100% | — ion molécul. + $H^+$ |
| 220 | 64% | — perte de COOH | 157 | | |
| 204 | 4% | | | | |
| 190 | 18% | | | | |
| 165 | 15% | | | | |
| 155 | 7% | — 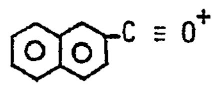 | | | |
| 138 | 21% | — 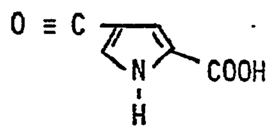 | | | |
| 127 | 46% | | | | |
| 120 | 100% | | | | |
| 92 | .26% | | | | |

### Après traitement Diazométhane

| Impact électronique | | | Ionisation chimique | | |
|---|---|---|---|---|---|
| 279 | 25% | — pic moléculaire | 297 | 10% | — ion molécul. + $NH_4^+$ |
| 246 | 8% | (-33) | 294 | 18% | — contamination précédent |
| 234 | 5% | | 280 | 100% | — ion molécul. + $H^+$ |
| 220 | 31% | | | | |
| 190 | 10% | | | | |
| 175 | 8% | | | | |
| 166 | 8% | | | | |
| 155 | 13% | | | | |
| 153 | 20% | | | | |
| 144 | 19% | | | | |
| 127 | 39% | | | | |
| 120 | 57% | | | | |
| 100 | 100% | | | | |

## TABLEAU IV

### VALEURS PRINCIPALES OBTENUES A PARTIR DES SPECTRES DE MASSE DU DERIVE N-DEMETHYLE SYNTHETIQUE

### Acide 4-(naphtoyl-1)-pyrrolyl-2-carboxylique

| Impact électronique | | | Ionisation chimique $NH_3$ | | |
|---|---|---|---|---|---|
| 265 | 57% | — pic moléculaire | 266 | 100% | — ion molécul. + $H^+$ |
| 246 | 23% | — perte $H_2O$ (-19) | 246 | 7% | — perte $H_2O$ |
| 230 | 7% | — (-35) | 220 | 28% | — perte COOH |
| 220 | 75% | — perte de COOH | | | |
| 204 | 5% | | | | |
| 190 | 16% | | | | |
| 165 | 6% | | | | |
| 155 | 3% | — 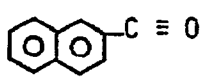 | | | |
| 138 | 8% | — 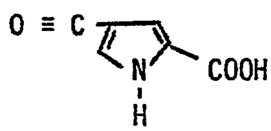 | | | |
| 127 | 24% | — naphtalène | | | |
| 120 | 100% | | | | |
| 95 | 28% | | | | |
| 69 | 90% | | | | |

### Après traitement Diazométhane

| | Ionisation chimique $NH_3$ | | |
|---|---|---|---|
| méthylation de la fonction acide ←— | 280 | 100% | — ion molécul. + $H^+$ |
| | 246 | 5% | |
| | 220 | 22% | |

EXEMPLE II

Caractérisation de l'acide N-méthyl-(5,6-dihydroxy-5,6-dihydronaphtoyl-1)-pyrrolyl-2-carboxylique.

TABLEAU V

VALEURS PRINCIPALES OBTENUES A PARTIR DES SPECTRES DE MASSE DU DERIVE (5,6-DIHYDROXY-5,6-DIHYDRONAPHTOYL-1)

Acide N-méthyl-(5,6-dihydroxy-5,6-dihydronaphtoyl-1)pyrrolyl-2-carboxylique

| Impact électronique | | | Ionisation chimique $NH_3$ | | |
|---|---|---|---|---|---|
| 313 | 10% | — pic moléculaire | 331 | 6% | — ion molécul. + $NH_4^+$ |
| 295 | 6% | — perte $H_2O$ | 314 | 100% | — pic molécul. + $H^+$ |
| 285 | 5% | — -28 ? | 296 | 6% | — perte $H_2O$ |
| 269 | 12% | — perte COOH | 280 | 4% | — perte O ? |
| 250)<br>251( | 6% | — perte COOH + $H_2O$ | 270 | 8% | — perte COOH |
| | | | 214 | 15% | |
| 240 | 3% | | 154 | 7% | |
| 222 | 6% | | | | |
| 206 | 2% | | | | |
| 184 | 25% | | | | |
| 152 | 30% | — 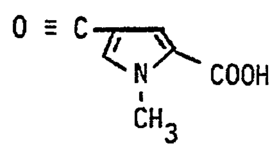 | | | |
| 115 | 30% | | | | |
| 115 | 30% | | | | |
| 108 | 40% | | | | |
| 58 | 100% | — pic de base | | | |

Après traitement Diazométhane

| Impact électronique | | |
|---|---|---|
| 327 | 65% | — Acide + $CH_3$ ⟹ ester méthylique |
| 309 | 5% | — perte $H_2O$ |
| 299 | 30% | |
| 283 | 22% | |
| 250 | 20% | |
| 240 | 30% | |
| 166 | 100% | — pic de base |

TABLEAU VI

SPECTRE RMN DANS LE DIMETHYLSULFOXYDE DU DERIVE (5,6-DIHYDROXY-5,6-DIHYDRONAPHTOYL-1) ET DU DERIVE (3,4-DIHYDROXY-3,4-DIHYDRONAPHTOYL-1) OBTENUS APRES EXTRACTION

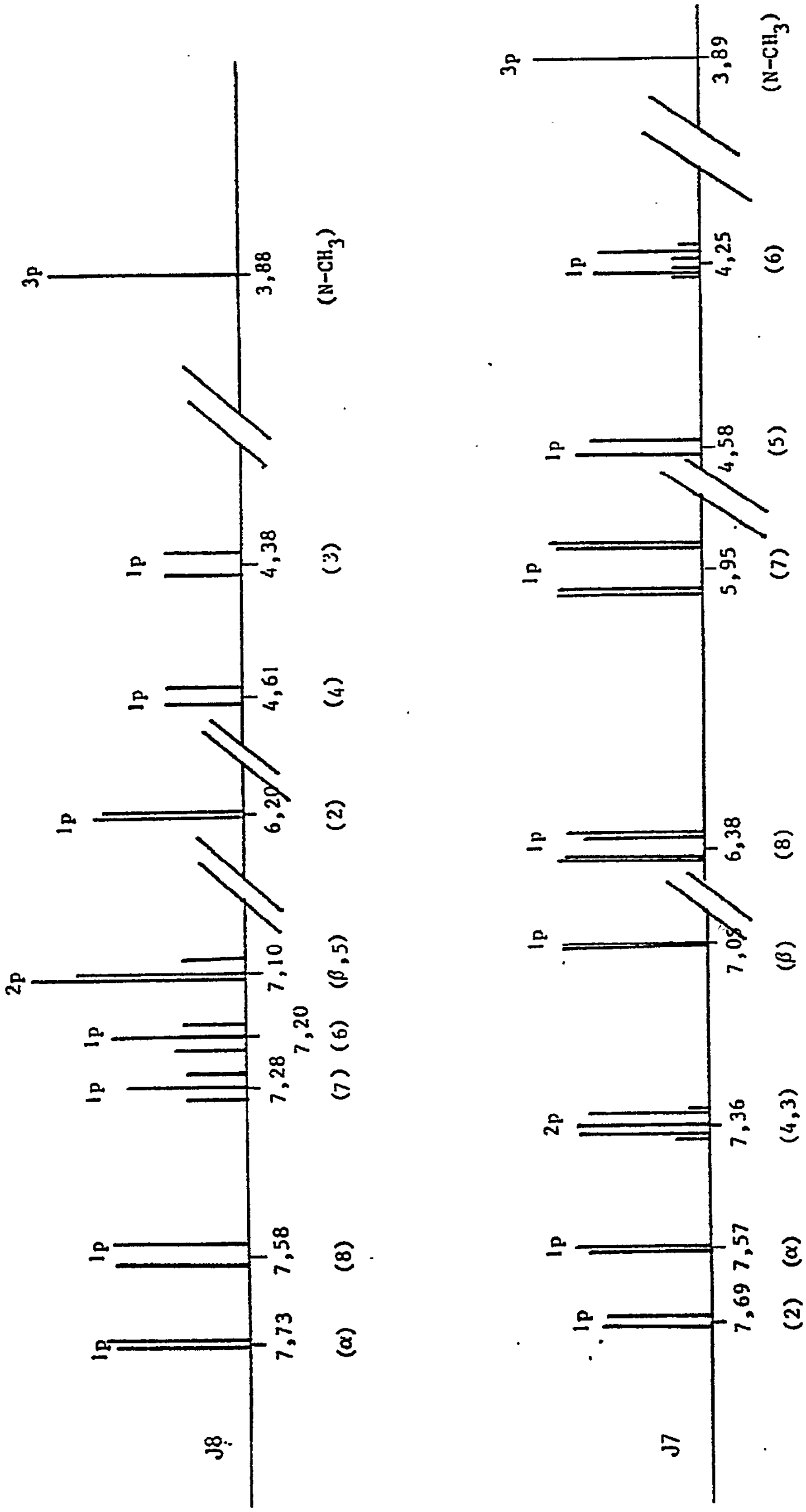

EXEMPLE III

Acide N-méthyl-(3,4-dihydroxy-3,4-dihydronaphtoyl-1)-pyrrolyl-2-carboxylique.

TABLEAU VII

VALEURS PRINCIPALES OBTENUES A PARTIR DES SPECTRES DE MASSE DU DERIVE N-METHYL-(3,4-DIHYDROXY-3,4-DIHYDRONAPHTOYL-1)-PYRROLYL-2-CARBOXYLIQUE

**Impact électronique**

| | | |
|---|---|---|
| 313 | 15% | — pic moléculaire |
| 295 | 12% | — perte $H_2O$ |
| 279 | 5% | — -34 |
| 269 | 7% | — perte COOH |
| 250 | 5% | |
| 234 | 8% | |
| 222 | 5% | |
| 206 | 2% | |
| 194 | 8% | |
| 170 | 2% | |
| 152 | 100% | — 0 ≡ C 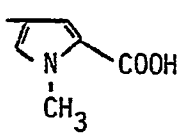 |
| 134 | 8% | |
| 131 | 15% | |
| 115 | 30% | |
| 108 | 70% | |
| 103 | 20% | |
| 58 | 40% | |

**Ionisation chimique $NH_3$**

| | | |
|---|---|---|
| 314 | 32% | — ion molécul. + H |
| 296 | 65% | — perte $H_2O$ |
| 280 | 8% | |
| 270 | 24% | |
| 252 | 100% | — pic de base |
| 152 | 19% | — 0 ≡ C 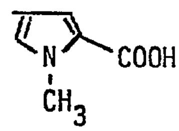 |

**Après traitement Diazométhane**

**Impact électronique**

| | | |
|---|---|---|
| 327 | 14% | — Acide + $CH_3$ ⟹ ester méthylique |
| 309 | 16% | — perte $H_2O$ |
| 250 | 16% | |
| 166 | 100% | |
| 152 | 10% | |

EXEMPLE IV

Acide N-méthyl-4-(3-hydroxy-naphtoyl-1)-pyrrolyl-2-carboxylique et Acide N-méthyl-4-(5-hydroxy-naphtoyl-1)-pyrrolyl-2-carboxylique.

TABLEAU VIII

VALEURS PRINCIPALES OBTENUES A PARTIR DES SPECTRES DE MASSE DU DERIVE N-METHYL-(3-HYDROXYNAPHTOYL-1)-PYRROLYL-2-CARBOXYLIQUE OBTENU PAR EXTRACTION

| Impact électronique | Ionisation chimique |
|---|---|
| Pas de masses supérieures à 100 | 296 100% — ion molécul. + $H^+$ |
| | 252 60% — perte COOH |

Après traitement Diazométhane

| Impact électronique | Ionisation chimique |
|---|---|
| 323 54% — diméthyl | 324 100% — diméthyl + $H^+$ |
| 306 6% — perte (-17) | |
| 292 6% — perte $OCH_3$ | |
| 264 63% — perte $COOCH_3$ | |
| 195 4% | |
| 185 8% | |
| 166 100% | |
| 134 31% | |
| 114 20% | |
| 99 53% | |

## TABLEAU IX

VALEURS PRINCIPALES OBTENUES A PARTIR DES SPECTRES DE MASSE DU DERIVE N-METHYL-4-(5-HYDROXYNAPHTOYL-1)-PYRROLYL-2-CARBOXYLIQUE OBTENU PAR EXTRACTION

| Impact électronique | | | Ionisation chimique |
|---|---|---|---|
| 295 | 1,9% | —— pic moléculaire | |
| 281 | 0,4% | —— perte $CH_3$ | |
| 279 | 1,2% | —— perte O | |
| 278 | 0,6% | —— perte $H_2O$ | |
| 250 | 2,2% | —— perte COOH | |
| 234 | 0,8% | —— (250 - 0) | |
| 220 | 2,0% | | |
| 211 | 4,0% | | |
| 180 | 1,2% | | |
| 172 | 3,4% | | |
| 166 | 1,9% | | |
| 152 | 4,0% | | |
| 122 | 21% | | |
| 105 | 31% | | |
| 99 | 30% | | |
| 91 | 23% | | |
| 86 | 24% | | |
| 84 | 46% | | |
| 77 | 26% | | |
| 58 | 100% | | |

Après traitement Diazométhane

| Impact électronique | | | Ionisation chimique | | |
|---|---|---|---|---|---|
| 323 | 16% | —— diméthyl | 324 | 98% | —— diméthyl + $H^+$ |
| 309 | 48% | —— monométhyl | 310 | 100% | —— monométhyl + $H^+$ |
| 292 | 16% | | 280 | 30% | ? |
| 264 | 12% | | | | |
| 250 | 50% | | | | |
| 180 | 21% | | | | |
| 166 | 100% | | | | |

TABLEAU X

COMPARAISON DES SPECTRES RMN DANS LE DIMETHYLSULFOXYDE DES DERIVES (3-HYDROXY OU 5-HYDROXY-NAPHTOYL-1) AVEC LE (4-HYDROXY-NAPHTOYL-1) DE SYNTHESE

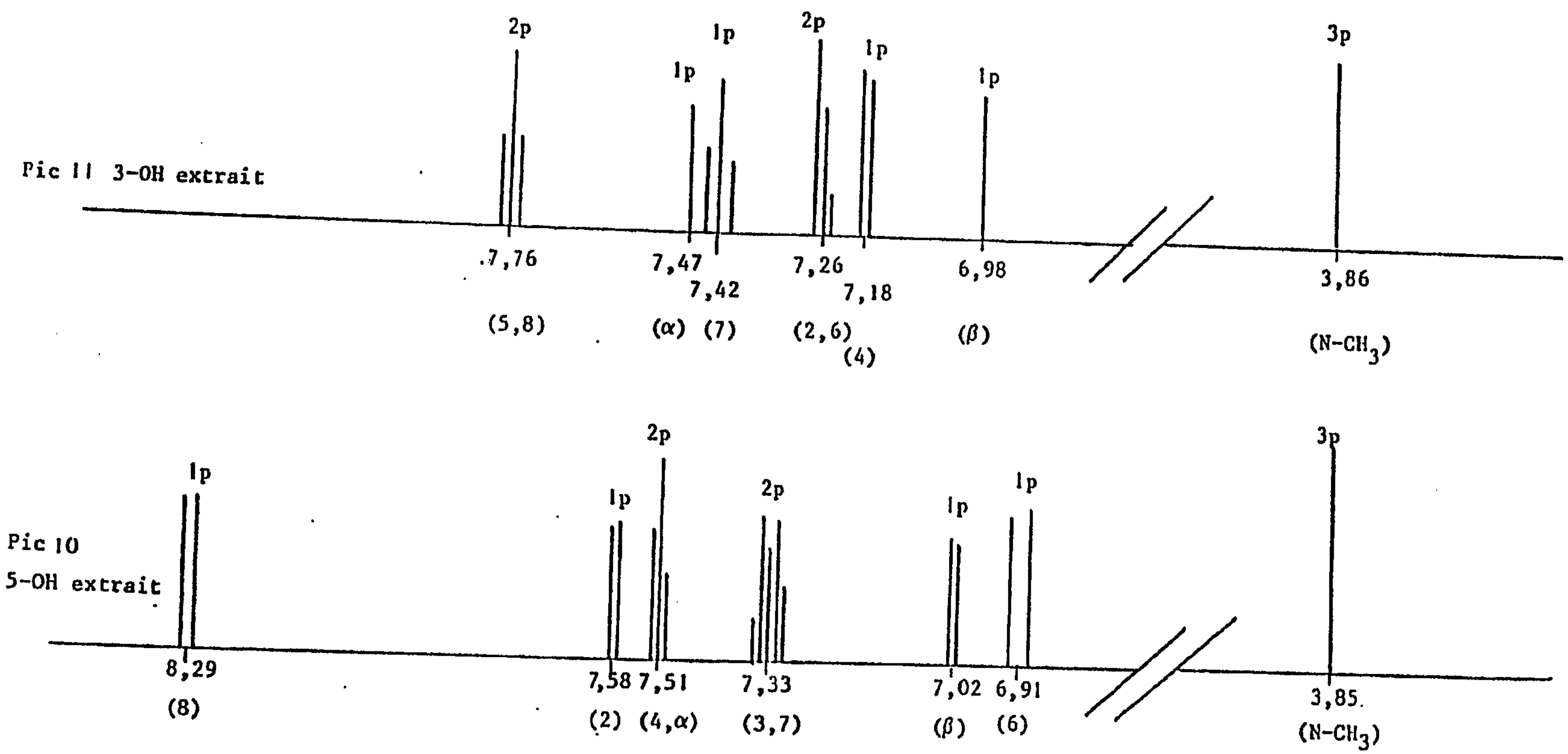

EXEMPLE V

Acide N-méthyl-4-(3-méthylsulfinylnaphtoyl-1)-pyrrolyl-2-carboxylique et Acide N-méthyl-4-(6-méthylsulfinylnaphtoyl-1)-pyrrolyl-2-carboxylique.

TABLEAU XI

VALEURS PRINCIPALES OBTENUES A PARTIR DES SPECTRES DE MASSE DU COMPOSE N-METHYL-4-(3-METHYLSULFINYLNAPHTOYL-1)-PYRROLYL-2-CARBOXYLIQUE OBTENU PAR EXTRACTION PAR HPLC

| Impact électronique | | | Ionisation chimique $NH_3$ | | |
|---|---|---|---|---|---|
| 341 | 10% | — pic moléculaire | 359 | 15% | — ion molécul. + $NH_4^+$ |
| 325 | 41% | — (perte - O) | 342 | 100% | — pic molécul. + $H^+$ |
| 297 | 7% | — (perte - COOH) | 326 | 30% | — (perte O) |
| 281 | 38% | | 298 | 10% | — (perte COOH) |
| 211 | 11% | | 282 | 10% | — (298 - O) |
| 205 | 7% | | 267 | 4% | — (298 - $OCH_3$) |
| 167 | 26% | | 236 | 3% | |
| 152 | 100% | — O ≡ C 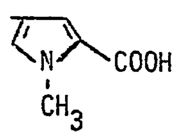 | 223 | 4% | |
| | | | 205 | 2% | |
| | | | 191 | 6% | — (naphtalène + $SOCH_3$) |
| 129 | 34% | | 152 | 6% | |
| 108 | 79% | | | | |
| 91 | 36% | | | | |

Après traitement Diazométhane

| Impact électronique | | | Ionisation chimique $NH_3$ | | |
|---|---|---|---|---|---|
| 355 | 12% | — pic moléculaire | 356 | 100% | — ion molécul. + $H^+$ |
| 340 | 12% | — (perte $CH_3$) | 342 | 70% | — (perte $CH_3$) |
| 324 | 1% | — (perte O + $CH_3$) | 340 | 80% | — (perte - O) |
| 308 | 1% | | 326 | 20% | (perte O + $CH_3$) |
| 296 | 1% | — (340 - COOH) | 324 | 10% | — (perte 2 O) |
| 282 | 1% | | 298 | 10% | |
| 223 | 2% | | 294 | 15% | — (perte $SOCH_3$ ?) |
| 166 | 30% | | 282 | 8% | |
| 163 | 2% | | 191 | 6% | |
| 113 | 40% | | 155 | 20% | |
| 104 | 25% | | | | |
| 83 | 30% | | | | |
| 59 | 100% | | | | |

TABLEAU XII

SPECTRE RMN DANS LE DIMETHYLSULFOXYDE DES DERIVES (3-METHYLSULFINYL)- ET (6-METHYLSULFINYL-NAPHTOYL-1) EXTRAITS PAR HPLC

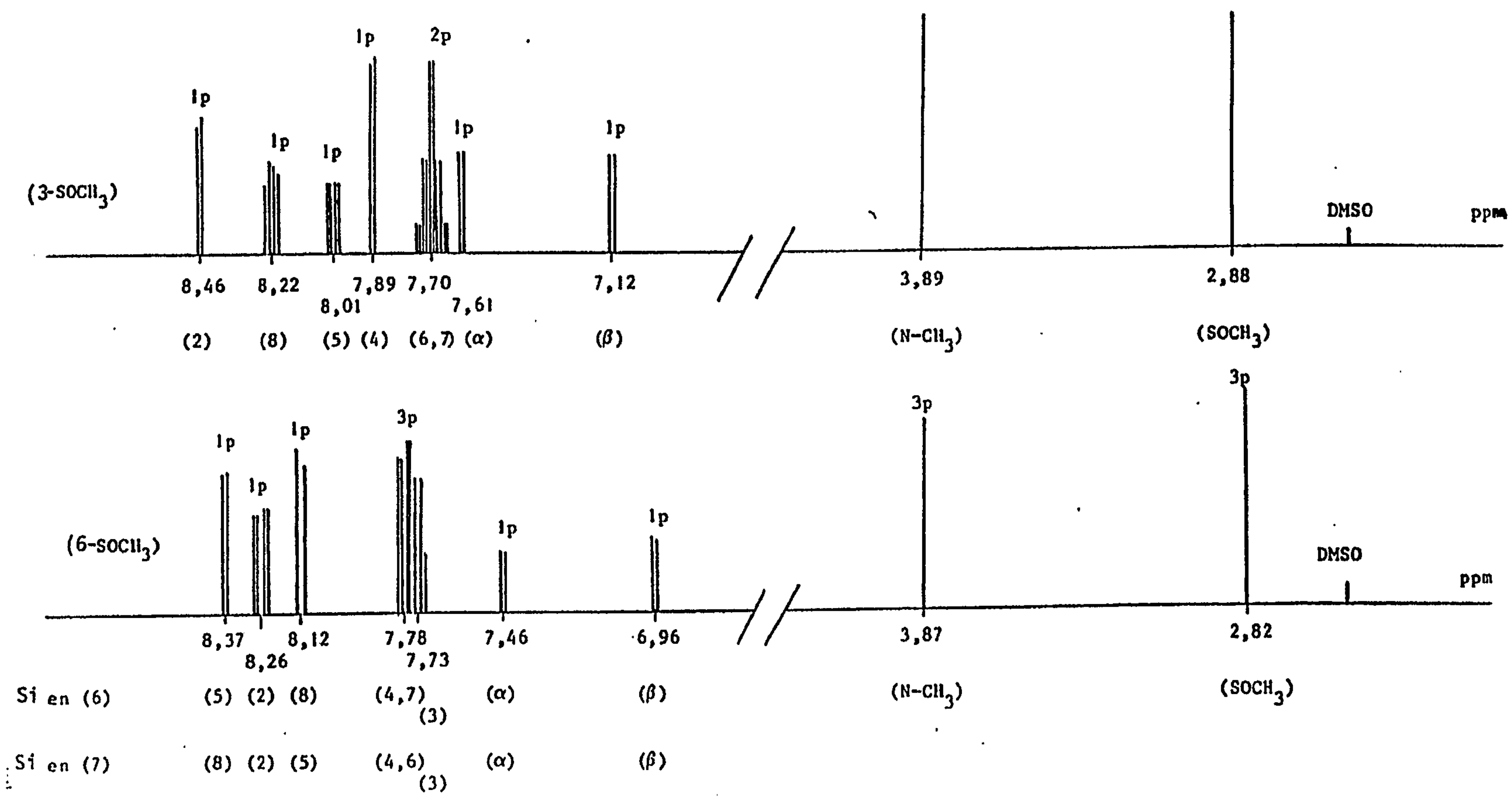

TABLEAU XIII

COUPLAGES OBSERVES APRES IRRADIATIONS SUCCESSIVES A (8,46), (8,22), (8,01) ppm ET INTERPRETATION DE LA PARTIE AROMATIQUE DU SPECTRE RMN DU DERIVE (3-METHYLSULFINYL-NAPHTOYL-1) OBTENU PAR HPLC

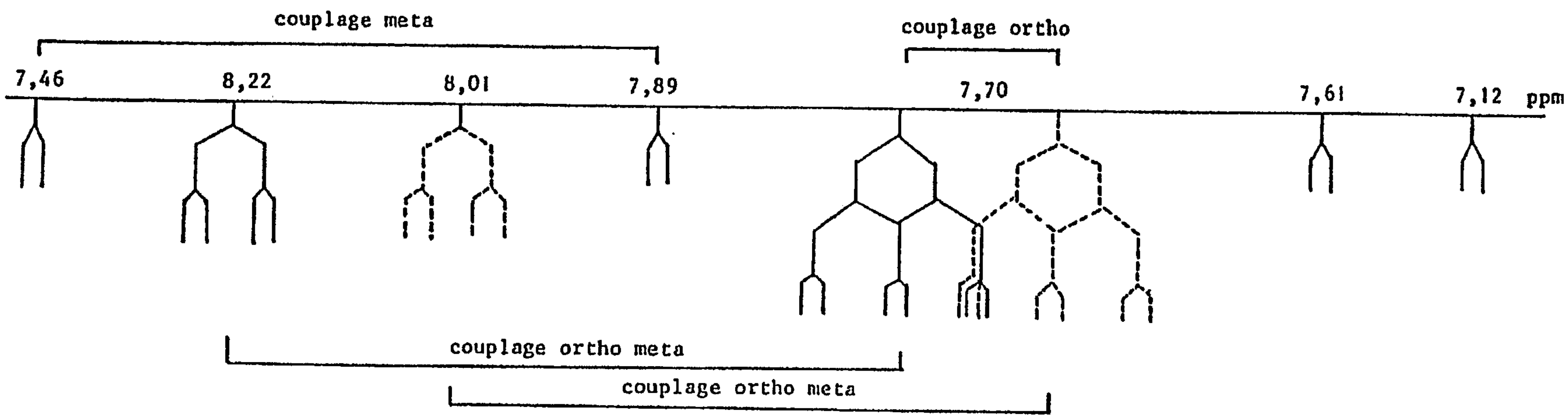

## TABLEAU XIV

VALEURS PRINCIPALES OBTENUES A PARTIR DES SPECTRES DE MASSE DU DERIVE (6-METHYLSULFINYLNAPHTOYL-1)

| Impact électronique | | | Ionisation chimique | | |
|---|---|---|---|---|---|
| 341 | 7% | —— pic moléculaire | 359 | 2% | —— ion molécul. + $NH_4^+$ |
| 325 | 12% | —— (perte 1 ox.) | 342 | 100% | —— ion molécul. + $H^+$ |
| 281 | 16% | —— (325 - COOH) | 326 | 7% | —— (perte $CH_3$) |
| 267 | 5% | —— (281 - $CH_3$) | 298 | 6% | —— (perte COOH) |
| 152 | 95% | —— O ≡ C 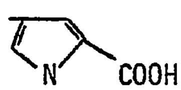 | 282 | 6% | —— (298 - 1 ox.) |
| | | | 267 | 12% | —— (298 - $OCH_3$) |
| 129 | 50% | | 223 | 6% | |
| 108 | 41% | | 205 | 2% | |
| 105 | 100% | | 191 | 5% | —— (naphtalène - $SOCH_3$ ?) |
| | | | 152 | 3% | |

### Après traitement Diazométhane

| | | | | | |
|---|---|---|---|---|---|
| 355 | 2% | —— pic moléculaire | 373 | 7% | —— ion molécul. + $NH_4^+$ |
| 340 | 3% | —— (perte $CH_3$) | 356 | 100% | —— ion molécul. + $H^+$ |
| 162 | 1% | | 340 | 35% | —— (perte -O) |
| 132 | 8% | | 294 | 3% | (perte O + O) |
| 113 | 20% | | 282 | 3% | —— (perte $SOCH_3$ ?) |
| 83 | 15% | | 261 | 2% | |
| 71 | 100% | | 167 | 1% | |
| 70 | 95% | | 155 | 6% | |

## TABLEAU XV

COUPLAGES OBSERVES APRES IRRADIATIONS SUCCESSIVES A (8,46), (8,22), (8,01) ppm ET INTERPRETATION DE LA PARTIE AROMATIQUE DU SPECTRE RMN DU DERIVE 4-(6-METHYLSULFINYL-NAPHTOYL-1)-N-METHYL-PYRROLYL-2-CARBOXYLIQUE

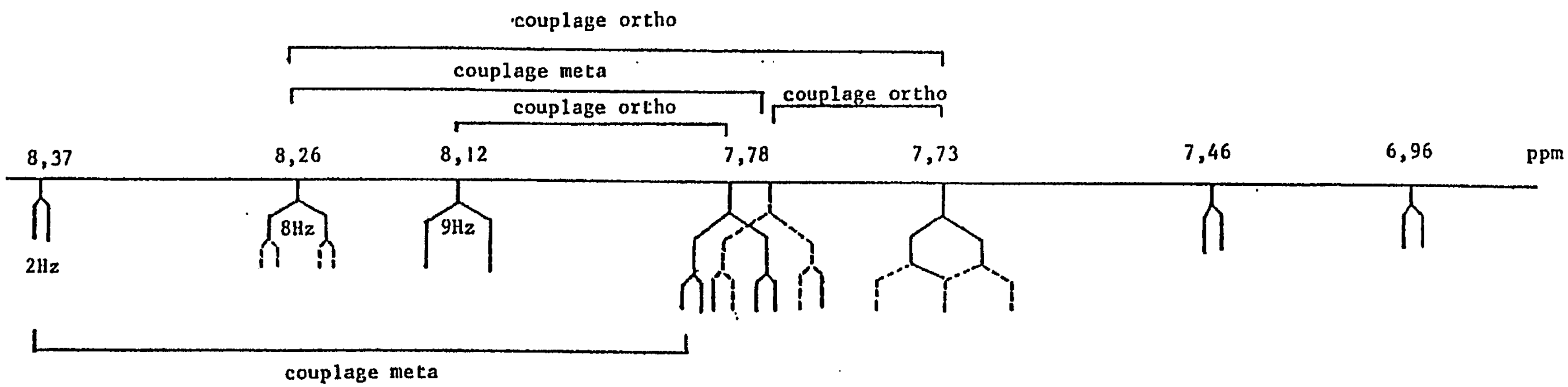

EXEMPLE VI

Etude des composés selon l'invention en tant qu'immuno-suppresseurs.

On a choisi comme éléments de comparaison l'Azathioprine et l'acide N-méthyl-4-(naphtoyl-1)-pyrrolyl-2-carboxylique.

A) Activité des composés selon l'invention sur le test des rosettes in vitro

L'étude a été réalisée sur globules rouges de mouton avec des cellules spléniques de souris $C_{57}BL/6J$. La modulation de la formation des rosettes par les composés selon l'invention par rapport à l'Azathioprine, a abouti aux résultats suivants:

1.- l'Azathioprine inhibe la formation des rosettes à des concentrations allant de 1 à 10 µg/ml.

2.- L'acide N-méthyl-4-(naphtoyl-1)-pyrrolyl-2-carboxylique inhibe la formation des rosettes dans les mêmes proportions, à des concentrations allant aussi de 1 à 10 µg/ml.

3.- L'acide 4-(naphtoyl-1)-pyrrolyl-2-carboxylique possède une activité inhibitrice du même ordre que celle du dérivé N-méthylé.

4.- le dérivé N-méthyl-5-hydroxylé est également inhibiteur à un degré identique, mais il possède, en plus, une activité immuno-stimulante à faible concentration.

5.- L'acide N-méthyl-4-(5,6-dihydroxy-5,6-dihydronaphtoyl-1)-pyrrolyl-2-carboxylique et l'acide N-méthyl-4-(3-méthylsulfinylnaphtoyl-1)-pyrrolyl-2-carboxylique ont une activité comparable à celle de l'acide N-méthyl-4-(naphtoyl-1)-pyrrolyl-2-carboxylique.

6.- Le dérivé N-méthyl-(3-hydroxy-naphtoyl-1) possède une activité mixte immuno-stimulante aux concentrations allant de 1 à 10 µg/ml.

En conclusion, les dérivés de l'acide N-méthyl-4-naphtoyl-pyrrolyl-2-carboxylique modulent la formation des rosettes in vitro à des concentrations voisines de celles de l'Azothioprine. Le dérivé N-méthyl-4-(3-hydroxy-naphtoyl-1)-pyrrolyl-2-carboxylique présente la particularité de stimuler la formation des rosettes.

B) Activité des composés selon l'invention sur le test de transformation lymphoblastique

Sur le test de transformation lymphoblastique, les composés selon l'invention montrent une activité antimitotique, aux doses allant de 5 à 50 µg/ml. Pour certains composés comme par exemple le dérivé 3-méthylsulfinyle et le dérivé N-déméthylé, une activité stimulante est par ailleurs notée à faible concentration, aussi bien vis-à-vis de la Concanavaline A que par rapport au LPS (Lipopolysaccharides de E. Coli). La mise en évidence d'une activité immuno-stimulante pour certains de ces composés permet d'envisager une autre application thérapeutique.

## Revendications

1. Dérivés naphtoylés de l'acide pyrrolyl-2-carboxylique répondant à la formule générale I:

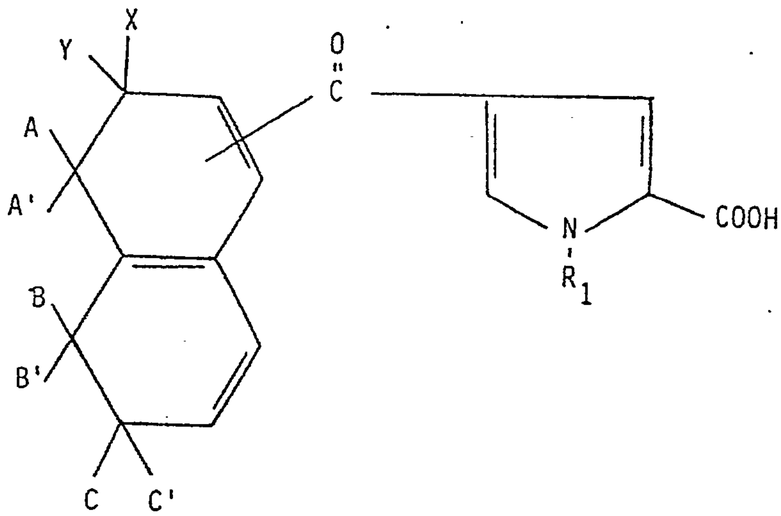

(I)

dans laquelle:
$R_1$ représente un radical méthyle, un radical phényle ou de l'hydrogène;
X est de l'hydrogène, un hydroxyle ou le groupe méthylsulfinyle;
Y est de l'hydrogène ou forme avec A' une double liaison;
A est de l'hydrogène ou un hydroxyle;
A' est de l'hydrogène ou forme avec Y une double liaison;
B est de l'hydrogène ou un hydroxyle;
B' est de l'hydrogène ou forme avec C une double liaison;
C est de l'hydrogène ou forme avec B' une double liaison;
C' est de l'hydrogène, un hydroxyle ou le groupe méthylsulfinyle ($SOCH_3$);
avec la restriction que X, A, B et C' ne représentent pas simultanément de l'hydrogène lorsque A' forme avec Y une double liaison et lorsque B' forme avec C une double liaison.

2. Les sels des composés de formule générale I avec une base minérale ou organique.

3. Un composé selon la revendication 1, répondant à la formule générale $I_A$:

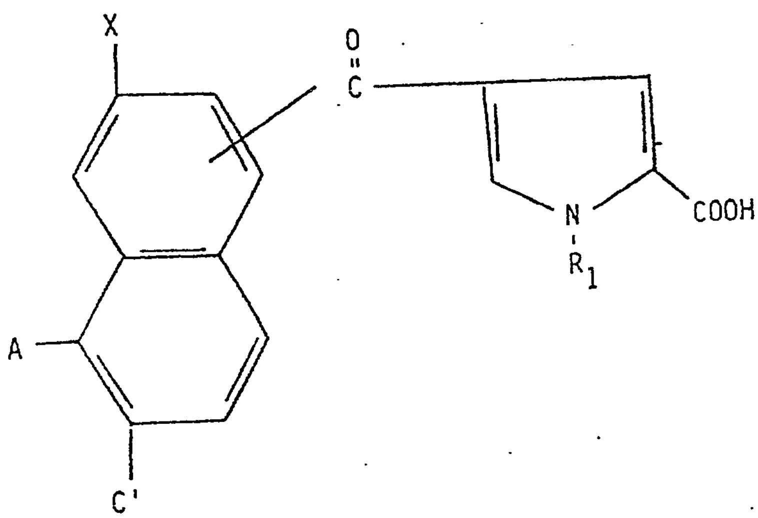

$(I_A)$

dans laquelle:
$R_1$ est défini comme précédemment;
X est un groupe méthylsulfinyle et C' est de l'hydrogène ou X est de l'hydrogène et C' est un groupe méthylsulfinyle et
A est de l'hydrogène ou un hydroxyle.

4. Un composé selon la revendication 1, répondant à la formule générale $I_B$:

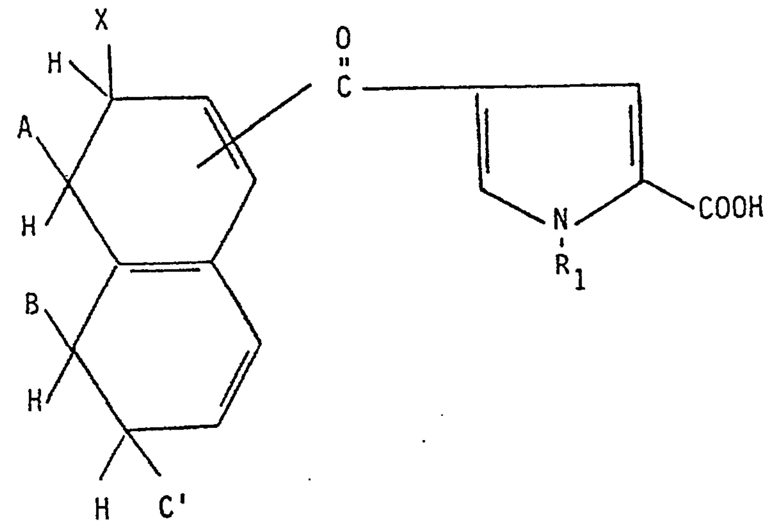

($I_B$)

dans laquelle:
X est un hydroxyle et A est un hydroxyle;
B et C′ sont de l'hydrogène; ou B et C′ sont chacun un hydroxyle et X et A sont de l'hydrogène; et
$R_1$ est défini comme précédemment.

5. Un composé selon la revendication 1, répondant à la formule générale $I_C$:

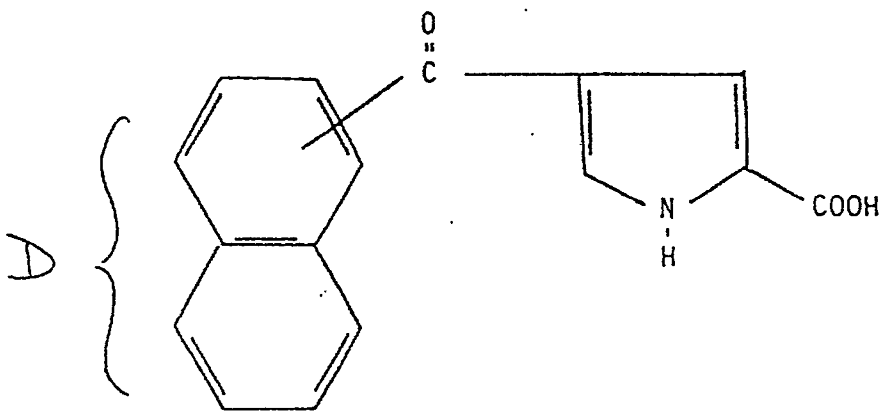

($I_C$)

dans laquelle D représente de l'hydrogène, un hydroxyle ou un méthylsulfinyle.

6. L'acide N-méthyl-4-(3-méthylsulfinylnaphtoyl-1)-pyrrolyl-2-carboxylique selon la revendication 1.

7. L'acide N-méthyl-4-(3-hydroxy-naphtoyl-1)-pyrrolyl-2-carboxylique selon la revendication 1.

8. L'acide N-méthyl-4-(5-hydroxy-naphtoyl-1)-pyrrolyl-2-carboxylique selon la revendication 1.

9. Les compositions pharmaceutiques renfermant à titre de principe actif au moins un composé selon l'une des revendications 1 à 8, en association ou en mélange avec un véhicule ou un excipient inerte non toxique pharmaceutiquement compatible.

10. Une composition pharmaceutique selon la revendication 9, dans laquelle l'excipient ou le véhicule est un de ceux qui conviennent pour l'administration par voie parentérale, digestive, rectale ou topique.

**Patentansprüche**

1. Die Naphtoyl Derivate der Pyrrolyl-2 Carbonsäure mit der allgemeinen Formel I

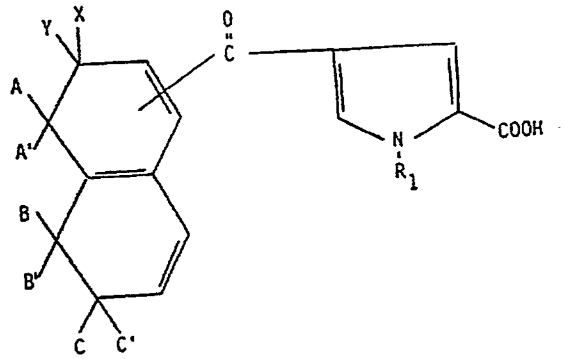

(I)

worin
$R_1$ ein Methyl, ein Phenyl oder ein Wasserstoff Atom bedeutet
X ein Wasserstoff Atom, ein Hydroxyl oder ein Methylsulfinyl Rest ist
Y ein Wasserstoff Atom ist, oder zusammen mit A′ eine Doppelbindung ist.
A ein Wasserstoff Atom oder ein Hydroxyl ist
A′ ein Wasserstoff Atom oder zusammen mit Y eine Doppelbindung ist
B′ ein Wasserstoff Atom oder ein Hydroxyl ist
B′ ein wasserstoff Atom oder zusammen mit C ein Doppelbindung ist
C ein Wasserstoff Atom oder zusammen mit B′ eine Doppelbindung ist
C′ ein Wasserstoff Atom, ein Hydroxyl oder ein Methylsulfinyl ($SOCH_3$) Rest ist mit der Ausnahme, dass X, A, B und C′ nicht gleichzeitig Wasserstoff bedeuten, als A′ zusammen mit Y eine Doppelbindung bildet und als B′ zusammen mit C, eine Doppelbindung bildet.

2. Die Salze der Verbindungen der allgemeinen Formel I mit einer anorganischen oder organischen Base.

3. Eine Verbindung nach Anspruch 1, entsprechend der allgemeinen Formel $I_A$

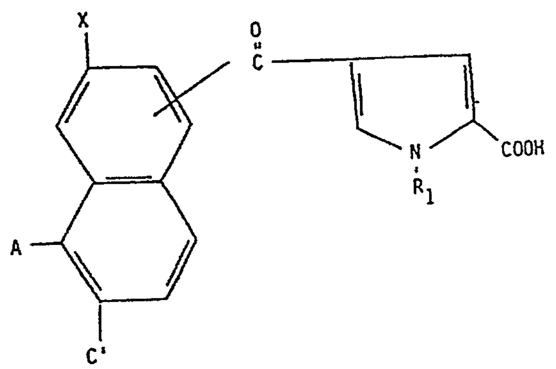

($I_A$)

worin
$R_1$ wie vorher definiert ist
X ein Methylsulfinyl Rest und
C′ ein Wasserstoff Atom ist, oder
X ein Wasserstoffatom und C′ ein Methylsulfinyl Rest ist, und
A ein Wasserstoff Atom oder ein Hydroxyl ist.

4. Eine Verbindung nach Anspruch 1 entsprechend der allgemeinen Formel $I_B$

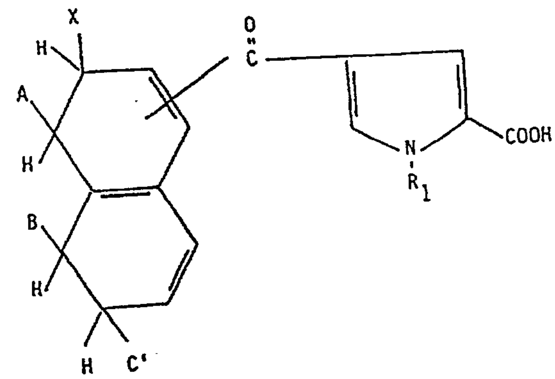

($I_B$)

worin
X ein Hydroxy und A ein Hydroxy sind
B und C' Wasserstoff sind oder
B und C' jedes ein Hydroxy sind und X und A Wasserstoff bedeuten und $R_1$ wie vorher definiert ist

5. Eine Verbindung nach Anspruch 1, entsprechend der allgemeinen Formel $I_C$

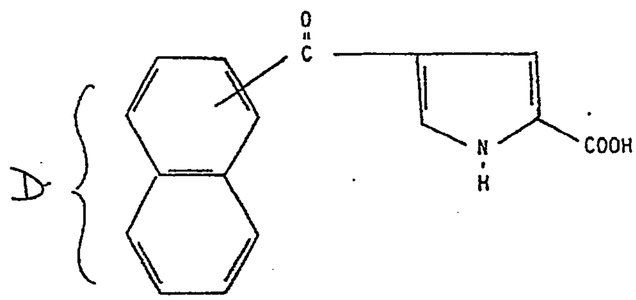

($I_C$)

worin
D ein Wasserstoff Atom, ein Hydroxy oder ein Methylsulfinyl Rest ist.

6. [(N-Methyl 4-(3-Methylsulfinyl) Naphtoyl-1) pyrrolyl-2] Carbonsäure nach Anspruch.1.

7. [N-Methyl 4-(3-hydroxy Naphtoyl-1) pyrrolyl-2]Carbonsäure nach Anspruch 1.

8. [N-Methyl 4-(5-hydroxy Naphtoyl-1) pyrrolyl-2] Carbonsäure nach Anspruch 1.

9. Pharmazeutische Zubereitungen die als Wirkstoff mindestens eine Verbindung nach einer der Ansprüche 1 bis 8 enthalten, in Verbindung oder Vermischung mit einem inerten, ungiftigen, pharmazeutisch verträglichen Vehikel oder Träger.

10. Eine pharmazeutische Zubereitung nach Anspruch 9, in der der Träger oder das Vehikel ein unter der die für die parenterale, digestive, rektale oder topische Verabreichung, geeignet sind.

## Claims

1. The Naphtoyl derivatives of pyrrolyl-2 carboxylic acid having the general formula I.

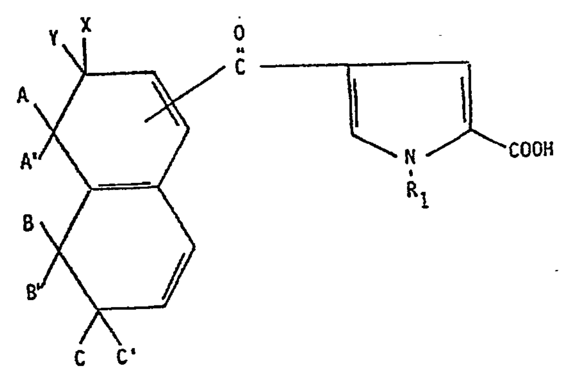

(I)

Wherein

$R_1$ is the methyl radical, the phenyl radical or a hydrogen
X is a hydrogen, a hydroxy or a methylsulfinyl radical
Y is a hydrogen, or together with A is a double bond
A is a hydrogen or a hydroxy
A' is a hydrogen or together with Y is a double bond
B is a hydrogen or a hydroxy
B' is a hydrogen or together with C is a double bond
C is a hydrogen or together with B' is a double bond
C' is a hydrogen, a hydroxy or a methylsulfinyl group.
With the proviso that X, A, B and C are not at the same time a hydrogen when A' together with Y is a double bond and when B' together with C is a double bond.

2. The base addition salts of the compounds of general formula I with a mineral or organic base.

3. A compound according to claim 1 having the general formula $I_A$

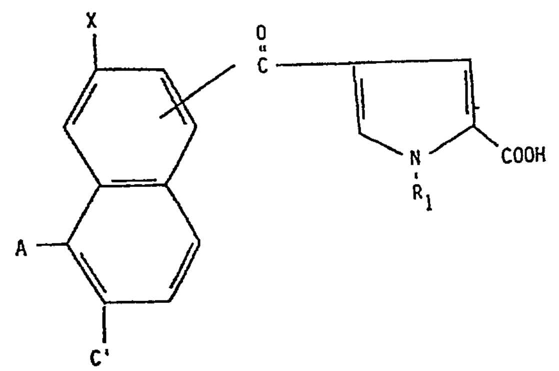

($I_A$)

Wherein
$R_1$ is defined as a previously-given
X is a methylsulfinyl group and C is a hydrogen
or X is a hydrogen and C' is a methylsulfinyl group
and A is a hydrogen or a hydroxy.

4. A compound according to claim 1 having the general formula $I_B$

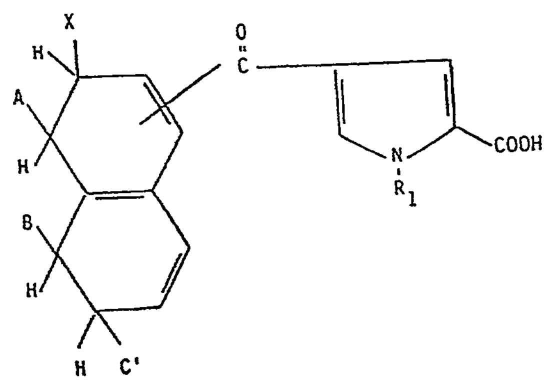

($I_B$)

Wherein
X is a hydroxy and A is a hydroxy, B and C' are hydrogen or B and C' are each a hydrogen atom and X and A are both a hydrogen atom and R is defined as previously-given.

5. A compound according to claim 1 having the general formula $I_C$

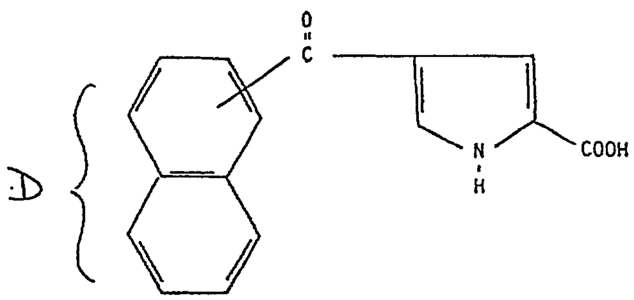

(Ic)

Wherein
D is a hydrogen, a hydroxy or a methylsulfinyl group.

6. [N-methyl 4-(3-methylsulfinyl naphtoyl-1) pyrrolyl-2] carboxylic acid according to claim 1.

7. [N-methyl 4-(3-hydroxy naphtoyl-1)pyrrolyl-2] carboxylic acid according to claim 1.

8. [N-methyl 4-(5-hydroxy naphtoyl-1)pyrrolyl-2] carboxylic acid according to claim 1.

9. Pharmaceutical compositions which contain as active ingredient at least one compound according to any of the claims 1 to 8, in admixture or conjunction with an inert non-toxic pharmaceutically-acceptable vehicle or carrier.

10. A pharmaceutical composition according to claim 9 in which the carrier or vehicle is one of those suitable for administration through the parenteral, digestive, rectal or topic routes of administration.